# EUROPEAN PATENT SPECIFICATION

(11) **EP 1 828 759 B1**
(45) Date of publication and mention of the grant of the patent: **15.12.2010**
(21) Application number: 05817169.5
(22) Date of filing: 05.10.2005
(51) Int. Cl.: G01N 27/327, C12Q 1/00

(54) **BIOSENSORS COMPRISING RUTHENIUM CONTAINING MEDIATORS AND METHOD OF USING THE SAME**
BIOSENSOREN MIT RUTHENIUM ENTHALTENDEN MEDIATOREN SOWIE VERFAHREN ZUR VERWENDUNG DAVON
BIOCAPTEURS COMPRENANT DES MEDIATEURS CONTENANT DU RUTHENIUM ET PROCEDE D'UTILISATION CORRESPONDANT

(30) Priority: 22.11.2004 US 629352 P
(43) Date of publication of application: 05.09.2007
(73) Proprietor: NIPRO DIAGNOSTICS, INC., Fort Lauderale, FL 33309 (US)
(72) Inventor: DENG, David, Z., Weston, FL 33327 (US); POPOVICH, Natasha, D., Pompano Beach, FL 33060 (US); HUNTER, Thomas, J., Cooper City, FL 33026 (US); SLOMSKI, Dennis, Wellington, FL 33467 (US); BELL, Douglas, Coral Springs, FL 33067 (US)
(74) Representative: Molnia, David
(86) International application number: PCT/US2005/036108
(87) International publication number: WO 2006/057722

(56) References cited:
- EP-A- 0 757 246
- WO-A-98/35232
- TETSU TATSUMA ET AL: "BIFUNCTIONAL LANGMUIR-BLODGETT FILM FOR ENZYME IMMOBILIZATION AND AMPEROMETRIC BIOSENSOR SENSITIZATION" THIN SOLID FILMS, ELSEVIER-SEQUOIA S.A. LAUSANNE, CH, vol. 202, no. 1, 15 July 1991 (1991-07-15), pages 145-150, XP000262139 ISSN: 0040-6090
- MORRIS N A ET AL: "AN ELECTROCHEMICAL CAPILLARY FILL DEVICE FOR THE ANALYSIS OF GLUCOSE INCORPORATING GLUCOSE OXIDASE AND RUTHENIUM(III) HEXAMINE AS MEDIATOR" ELECTROANALYSIS, VHC PUBLISHERS, INC, US, vol. 4, no. 1, 1992, pages 1-9, XP008060679 ISSN: 1040-0397
- KOSELA E ET AL: "CHARGE MEDIATION BY RUTHENIUM POLY(PYRIDINE) COMPLEXES IN 'SECOND-GENERATION' GLUCOSE BIOSENSORS BASED ON CARBOXYMETHYLATED BETA-CYCLODEXTRIN POLYMER MEMBRANES" ANALYTICAL AND BIOANALYTICAL CHEMISTRY, XX, DE, vol. 373, no. 8, 2002, pages 724-734, XP008060683 ISSN: 1618-2642
- WANG J ET AL: "IMPROVED ALCOHOL BIOSENSOR BASED ON RUTHENIUM-DISPERSED CARBON PASTE ENZYME ELECTRODES" JOURNAL OF ELECTROANALYTICAL CHEMISTRY AND INTERFACIAL ELECTROCHEMISTRY, ELSEVIER, AMSTERDAM, NL, vol. 353, no. 1/2, 1993, pages 113-120, XP008060669 ISSN: 0022-0728
- TATSUMA T ET AL: "ENZYME MONOLAYER- AND BILAYER-MODIFIED ELECTRODES WITH DIAPHORASE AND DEHYDROGENASES" JOURNAL OF ELECTROANALYTICAL CHEMISTRY AND INTERFACIAL ELECTROCHEMISTRY, ELSEVIER, AMSTERDAM, NL, vol. 310, no. 1/2, 1991, pages 149-157, XP000995069 ISSN: 0022-0728
- BATTAGLINI F ET AL: "ENZYME CATALYSIS AT HYDROGEL-MODIFIED ELECTRODES WITH SOLUBLE REDOX MEDIATOR" JOURNAL OF THE AMERICAN SOCIETY OF BREWING CHEMISTS, AMERICAN SOCIETY OF BREWING CHEMISTS, ST PAUL, MN, US, vol. 90, no. 7, 1 January 1994 (1994-01-01), page 98S/99S, XP008060673 ISSN: 0361-0470
- DEGANI Y ET AL: "DIRECT ELECTRICAL COMMUNICATION BETWEEN CHEMICALLY MODIFIED ENZYMES AND METAL ELECTRODES. 2. METHODS FOR BONDING ELECTRON-TRANSFER RELAYS TO GLUCOSE OXIDASE AND D-AMINO-ACID OXIDASE" JOURNAL OF THE AMERICAN CHEMICAL SOCIETY, AMERICAN CHEMICAL SOCIETY, NEW YORK, US LNKD- DOI:10.1021/JA00216A040, vol. 110, no. 8, 1 January 1988 (1988-01-01), pages 2615-2620, XP008060672 ISSN: 0002-7863

## Description

The present disclosure relates to biosensors for measuring an analyte in a bodily fluid, such as blood, wherein the biosensor comprises unique electrodes, a unique electron mediator or combinations thereof. The present disclosure also provides methods of measuring analytes in bodily fluid.

Electrochemical sensors have long been used to detect and/or measure the presence of analytes in a fluid sample. In the most basic sense, electrochemical sensors comprise a reagent mixture containing at least an electron transfer agent (also referred to as an "electron mediator") and an analyte specific bio-catalytic protein, and one or more electrodes. Such sensors rely on electron transfer between the electron mediator and the electrode surfaces and function by measuring electrochemical redox reactions. When used in an electrochemical biosensor system or device, the electron transfer reactions are transformed into an electrical signal that correlates to the concentration of the analyte being measured in the fluid sample.

The use of such electrochemical sensors to detect analytes in bodily fluids, such as blood or blood derived products, tears, urine, and saliva, has become important, and in some cases, vital to maintain the health of certain individuals. For example, testing and controlling blood glucose for people with diabetes can reduce their risk of serious damage to the eyes, nerves and kidneys.

An exemplary electrochemical biosensor is described in U.S. Patent No. 6,743,635 ('635 patent). The '635 patent describes an electrochemical biosensor used to measure glucose level in a blood sample. The electrochemical biosensor system is comprised of a test strip and a meter. The test strip includes a sample chamber, a working electrode, a counter electrode and fill-detect electrodes. A reagent layer is disposed in the sample chamber. The reagent layer contains an enzyme specific for glucose, glucose oxidase, and a mediator, potassium ferricyanide. When a user applies a blood sample to the sample chamber on the test strip, the reagents react with the glucose in the blood sample and the meter applies a voltage to the electrodes to cause redox reactions. The meter measures the resulting current that flows between the working and counter electrodes and calculates the glucose level based on the current measurements.

Battaglini F & Calvo EJ, "Enzyme Catalysis at Hydrogel-modified Electrodes with Soluble Redox Mediator", J. Chem. Soc. Faraday Trans., 1994, 90(7), 987-995, disclose an amperometric biosensor for the measurement of glucose in a fluid comprising a platinum electrode and a reaction reagent system comprising a pentaamine-pyrazine-ruthenium complex and glucose oxidase.

Testu Tatsuma et al, "Biofunctional Langmuir-Blodgett Film for Enzyme Immobilization and Amperometric Biosensor Sensitization" thin Sold Films," Elsevier-Sequoia S.a. Lausanne, CH, vol. 202, no. 1, 15 July 1991, pages 145-150, disclose a sensor with a tin oxide electrode, wherein a Langmuir-Blodgett film consisting of octadecylamine as the binding site for the enzyme molecule, an amphiphilic ferrocene derivative as the electron mediator and octadecanol as the matrix for these molecules is deposited on said tin oxide electrode.

Existing glucose biosensors typically contain potassium ferricyanide as the electron mediator. While ferricyanide is suitable for electrochemical detection on certain electrodes, it does have its drawbacks. In particular, ferricyanide converts to ferrocyanide when exposed to moisture and/or high temperature. This produces an increasing blank which compromises the usable shelf-life of the biosensor. Ferricyanide also requires a higher applied potential for electrochemical detection that generates interference from electro-oxidizable species, such as acetaminophen, ascorbate or uric acid, which may also be present in bodily fluids.

A major benefit for using electrochemical biosensors such as those described in the '635 patent is that only a small amount of blood sample is required to perform the measurement. However, as the size of the blood sample necessary to perform the measurement is decreased, the sensitivity of the biosensor also decreases and the ability to measure the electron-transfer kinetics during the redox reactions on the electrode surfaces must be improved to ensure the accuracy of the measurement. Biosensors with more efficient electron-transfer kinetics will result in enhanced sensor performance and are thus highly desirable.

Accordingly, novel biosensors are desired that overcome the drawbacks of current electron mediators and improve upon existing electrochemical biosensor technologies so that measurements are more accurate.

### SUMMARY

Disclosed herein are biosensors as defined in the claims used for measuring analyte in a bodily fluid, such as blood, comprising unique electrodes, a unique electron mediator, or combinations thereof. In one embodiment, a biosensor exhibiting superior electron transfer kinetics is described that comprises at least one or more electrodes comprising a semiconducting material. Examples of semiconducting electrodes that may be used include without limitation tin oxide, indium oxide, titanium dioxide, manganese oxide, iron oxide, and zinc oxide, or combinations of these materials, such as zinc oxide or tin oxide doped with indium or indium oxide doped with zinc or tin.

In another embodiment, the biosensor's electron transfer kinetics may be improved using a biosensor comprising at least one or more electrodes comprising thin film carbon material.

Also disclosed herein is a biosensor in which improved properties result from the use of a ruthenium containing electron mediator, wherein the ruthenium containing mediator is ruthenium hexaamine (III) trichloride. In this embodiment, the electrodes may be comprised of a semiconducting material or thin film carbon as described hereinabove, or in the alternative, the electrodes may be comprised of other more traditional conducting electrode materials, such as metals, including without limitation gold, platinum, rhodium, palladium, silver, iridium, steel, metallorganics, and mixtures thereof.

Also disclosed is a method of measuring the concentration of an analyte in a fluid sample using the biosensors of the present invention. For example, in one embodiment, the method comprises contacting a biosensor comprising at least one or more electrodes, comprised of either a semiconducting material, a conducting material, or a thin film carbon with a fluid sample. The reaction reagent system of the biosensor also comprise a ruthenium hexaamine (III) trichloride containing electron mediator.

The method described herein further comprises detecting an electrical signal and measuring the electrical signal to thereby determine the concentration of an analyte in the fluid sample.

In accordance with these and other objects which will become apparent hereinafter, the instant invention will now be described with particular reference to the accompanying drawings.

### BRIEF DESCRIPTION OF THE DRAWINGS

Fig. 1 is a cyclic voltammogram associated with the use of a tin doped indium oxide (ITO) electrode with a ruthenium hexaamine electron mediator.

Fig. 2 is a cyclic voltammogram associated with the use of an ITO electrode with a ferricyanide electron mediator.

Fig. 3 is a cyclic voltammogram associated with the use of an zinc doped indium oxide (IZO) electrode with a ruthenium hexaamine electron mediator.

Fig. 4 is a cyclic voltammogram associated with the use of a thin carbon film electrode with a ruthenium hexaamine electron mediator.

Fig. 5 is a cyclic voltammogram associated with the use of a thin carbon film electrode with a ferricyanide electron mediator.

Fig. 6 is a cyclic voltammogram associated with the use of a thin carbon film electrode with a ferrocene carboxylic acid electron mediator.

Fig. 7 is a cyclic voltammogram associated with the use of a palladium thin film electrode prepared using a metalloorganic approach with a ruthenium hexaamine electron mediator.

Fig. 8 is a cyclic voltammogram associated with the use of a palladium thin film electrode prepared using a metalloorganic approach with a ferricyanide electron mediator.

Fig. 9 is an Atomic Force Microscope (AFM) image of an IZO/Au film according to the present disclosure.

Fig. 10 is an AFM image of a thin carbon film according to the present disclosure.

Fig. 11 is a graph showing dose response as a function of glucose concentration for biosensors using mediators comprising 100, 150, and 200mM ruthenium hexaamine (III) trichloride.

Fig. 12 a graph showing glucose values as a function of excitation voltage for a 100 mg/dL blood sample using a reagent formulation comprising a glucose oxidase and a ruthenium hexaamine (III) trichloride mediator.

Fig. 13 is a graph showing reaction kinetics of electrodes made with a ferricyanide electron mediator (13a) and with a ruthenium hexaamine (III) trichloride mediator (13b) on a carbon electrode at zero wait time (zero incubation time).

Fig. 14 is a graph showing dose response on gold and IZO electrodes having a chemistry solution containing glucose oxidase (GO).

Fig. 15 is a graph showing dose response on gold and IZO electrodes having a chemistry solution containing glucose dehydrogenase (GDH).

Table 1 is a summary of the cyclic voltammetry data.

### DETAILED DESCRIPTION OF THE INVENTION

In accordance with the present disclosure provided herein are electrochemical biosensors developed for measuring an analyte in a non-homogenous fluid sample, such as a bodily fluid chosen from blood, urine, saliva and tears. At a minimum, the biosensor includes at least one or more electrodes and a reaction reagent system comprising an electron mediator and an oxidation-reduction enzyme specific for the analyte to be measured, wherein the electron mediator comprises a ruthenium hexaamine (III) trichloride containing material,

As used herein, the phrase "working electrode" is an electrode at which the electrochemical oxidation and/or reduction reaction occurs, e.g., where the analyte, typically the electron mediator, is oxidized or reduced.

"Counter electrode" is an electrode paired with the working electrode. A current of equal magnitude and of opposite polarity to the working electrode passes through the counter electrode.

In accordance with another aspect of the present disclosure, provided herein are biosensors comprising unique electrode materials, including semiconducting and conducting materials. The conducting materials include traditional metals, as well as novel thin film carbon materials.

Examples of semiconducting material include without limitation, tin oxide, indium oxide, titanium dioxide, manganese oxide, iron oxide, and zinc oxide, any or all of which may be doped with another element. For example, zinc oxide or tin oxide may be doped with indium. Alternatively, indium oxide may be doped with zinc or tin.

It has been found that the use of a ruthenium containing electron mediator in combination with such semiconducting electrodes, or even with traditional conducting electrode materials results in biosensors that are more stable in the ambient environment and are less sensitive to exposure to moisture than current biosensors. This extends the usable shelf-life of the biosensor and reduces the bias attributed to the background increase during the shelf-life of the biosensor test strips. In addition, such biosensors have also markedly reduced interference due to electro-oxidizable species present in the biological samples since the ruthenium containing mediator has a very low electrochemical oxidation potential.

As stated, a biosensor comprising a ruthenium containing electron mediator may also be used with a traditional conducting electrode material. Non-limiting examples of the conducting material include metals chosen from gold, platinum, rhodium, palladium, silver, iridium, carbon, steel, metallorganics, and mixtures thereof. Alternatively, a biosensor comprising a ruthenium containing electron mediator may be used with thin film carbon electrodes.

As described previously, in different embodiments, at least one electrode may comprise a thin film carbon material. As used herein a "carbon material" is meant to encompass any allotrope of carbon, depending on the desire to have a conducting or semiconducting electrode. More specifically, an allotrope of carbon is meant to encompass the different molecular configurations of carbon, including without limitation diamond, lonsdaleite, (a hexagonal a polymorph of diamond), graphite, amorphous carbon, fullerene, and carbon nanotubes.

One example of an allotrope of carbon that is contemplated for use in the present invention as a semiconducting electrode is doped diamond, such as diamond doped with boron, nitrogen, or phosphorous.

Carbon can also take a form that is not precisely defined as an allotrope, such as conducting carbon in the form of carbon black, which is defined as any of various finely divided forms of carbon derived from the incomplete combustion of natural gas or petroleum oil. While carbon black is a colloidal substance consisting wholly or principally of amorphous carbon, it usually contains a certain amount of impurities, such as acidic or basic functional groups or other adsorbed by-products from the production processes, such as aromatic compounds.

In addition, when the electrodes comprise at least one conducting carbon material, the material may comprise sputtered carbon or screen printed carbon. When sputtered, the carbon electrode typically further comprises chromium (Cr) that is present in the seed layer to promote carbon adhesion to the substrate and increase conductivity of the film.

In another embodiment, the above-described electrodes further comprise an inert support material onto which a thin layer of the semiconducting, conducting or thin film carbon material is deposited. As used herein "thin film" is meant to encompass a range from 50 angstroms to 400 µm.

Non-limiting examples of the support material include polymeric or plastic materials, such as polyethylene terepthalate (PET), glycol-modified polyethylene terepthalate (PETG), polyvinyl chloride (PVC), polyurethanes, polyamides, polyimide, polycarbonates, polyesters, polystyrene, or copolymers of these polymers, as well as ceramics, such as such as oxides of silicon, titanium, tantalum and aluminum, and glass. In addition to the insulating properties, the particular support material is chosen based on temperature stability, and the desired mechanical properties, including flexibility, rigidity, and strength.

Also disclosed herein are methods of measuring the concentration of an analyte in a fluid sample using the electrochemical biosensors of the present invention. An exemplary method comprises the steps of contacting a biosensor with fluid, wherein the biosensor comprises at least one or more electrodes comprising semiconducting material or a conducting material and a reaction reagent system comprising a ruthenium hexaamine (III) trichloride containing electron mediator and an oxydo-reductase enzyme specific for an analyte;
Applying voltage across the electrodes;
Detecting the sample fluid as it fills the reaction chamber;
Applying an excitation potential across the electrodes;
Measuring the resulting current; and
Converting the measured current to the concentration of the analyte in the fluid sample. In one embodiment, the meter can be turned on by inserting the strip.

The electrochemical biosensors described herein can be used to monitor analyte concentration in a non-homogeneous bodily fluid, such as blood. Non-limiting examples of such analytes include analytes of glucose, cholesterol, lactate, osteoporosis, ketone, theophylline, and hemoglobin A1 c. The specific enzyme present in the fluid depends on the particular analyte for which the biosensor is designed to detect, where representative enzymes include: glucose oxidase, glucose dehydrogenase, cholesterol esterase, cholesterol oxidase, lipoprotein lipase, glycerol kinase, glycerol-3-phosphate oxidase, lactate oxidase, lactate dehydrogenase, pyruvate oxidase, alcohol oxidase, bilirubin oxidase, uricase, and the like.

In one embodiment where the analyte of interest is glucose, the enzyme component of the redox reagent system is a glucose oxidizing enzyme, such as glucose oxidase, PQQ-dependent glucose dehydrogenase and NAD-dependent glucose dehydrogenase.

The electrochemical biosensors described herein may all be used in a system for measuring glucose concentration in blood, as described in U.S. Patent No. 6,743,635 B2.

When used to measure analytes in blood, the reaction reagent system typically further comprises a red blood cell binding agent for capturing red blood cells. Such binding agents include lectins.

Depending on the analyte of interest, the reaction reagent system may include such optional ingredients as buffers, surfactants, and film forming polymers. Examples of buffers that can be used in the present invention include without limitation potassium phosphate, citrate, acetate, TRIS, HEPES, MOPS and MES buffers. In addition, typical surfactants include non-ionic surfactant such as Triton X-100^{®} and Surfynol^{®}, anionic surfactant and zwitterionic surfactant. Triton X-100^{®} (an alkyl phenoxy polyethoxy ethanol), and Surfynol^{®} are a family of detergents based on acetylenic diol chemistry. In addition, the reaction reagent system may optionally include wetting agents, such as organosilicone surfactants, including Silwet^{®} (a polyalkyleneoxide modified heptamethyltrisiloxane from GE Silicones).

The reaction reagent system further optionally comprises at least one polymeric binder material. Such materials are generally chosen from the group consisting of hydroxypropyl-methyl cellulose, sodium alginate, microcrystalline cellulose, polyethylene oxide, polyethylene glycol (PEG), polypyrrolidone, hydroxyethylcellulose, or polyvinyl alcohol.

It has been discovered that the use of certain optional ingredients can lead to reagent formulations containing Ru mediator that spread more uniformly and that are more tolerant of slight misalignment of dispense location. Such uniform spreading of reagent on the sensors tend to eliminate thicker deposition typically occuring on the edge of reagent deposition (referred to as the "coffee ring" or "igloo" effect). As a result, sensor repeatability or precision performance is improved and outlier strips due to uneven reagent deposition or misaligned deposition are reduced or eliminated. For example, formulation containing polyvinyl alcohol (PVA) and/or Natrosol (a hydroxyethylcellulose from Aqualon, a division of Hercules, Inc.) and Triton X-100 or Silwet will produce very uniform reagent spreading.

In one embodiment, a reagent formulation containing 1% Natrosol, 250L and 0.05% Triton-X 100 showed a precision performance of better than 4% at clinically important 75 mg/dL glucose level. In another embodiment, a reagent formulation containing 2% PVA and 0.15% Triton-X 100 showed better than 4% precision at all glucose levels. In addition, examination of sensors under magnification showed no misaligned deposition ('off-centered" reagent deposition).

For example, in one embodiment, 0.01 to 0.3%, such as 0.05 to 0.25% of a non-ionic surfactant such as Triton X-100 may be used in combination with 0.1 to 3%, such as 0.5 to 2.0% of a polymeric binder material, such as PVA.

Other optional components include dyes that do not interfere with the glucose reaction, but facilitates inspection of the deposition. In one non-limiting embodiment, a yellow dye (fluorescein) may be used.

In addition to the enzyme specific for the analyte and the electron mediator, the reaction reagent system mentioned above may also include the previously described optional components, including the buffering materials, the polymeric binders, and the surfactants. The reagent layer generally covers at least part of the working electrode as well as the counter electrode.

When used as an electrochemical blood glucose sensor, the chemical constituents in the reagent layer reacts with glucose in the blood sample in the following way. The enzyme, such as glucose oxidase, initiates a reaction that oxidizes the glucose to gluconic acid and reduces the electron mediator. For example, when used, ferricyanide is reduced to ferrocyanide. When ruthenium hexaamine [Ru(NH₃)₆]³⁺ is used, it is reduced to [Ru(NH₃)₆]²⁺. When an appropriate voltage is applied to the working electrode, relative to the counter electrode, the electron mediator is oxidized. For example, ferrocyanide is oxidized to ferricyanide, thereby generating a current that is related to the glucose concentration in the blood sample. When ruthenium hexaamine [Ru(NH₃)₆]²⁺ is used, it is oxidized to [Ru(NH₃)₆]³⁺. To determine the efficiency of such reactions, the following electrochemical analysis was performed.

### Electrode Materials and Evaluation

The electrode materials according to the present disclosure include thin films of: semiconductors; sputtered carbon; and metal films that may be deposited using vapor deposition techniques, such as sputtering, or which may be derived from metalloorganic compounds. Performance of these materials was compared with the performance of gold films, since gold is well established as an excellent electrode material.

The physical properties of these materials were evaluated using standard techniques, which included the following:

Conductivity measurement: Van der Paaw four point contact resistivity measurements were made to determine sheet resistance.

Electrochemical Performance: Cyclic voltammetry with phosphate buffer (background current), was used in conjunction with various electron mediators, including without limitation ferricyanide, ruthenium hexaamine and ferrocene carboxylic acid.

Film properties: Electron and optical microscopy was used to evaluate the film for uniformity and defects.

Morphology: Atomic force microscopy (AFM) was used to determine surface roughness of the electrode materials.

Crystallographic texture and crystalline : X-ray diffraction was used to determine crystal structure, particularly for semiconductor films.

### Experimental Procedure For Cyclic Voltammetry

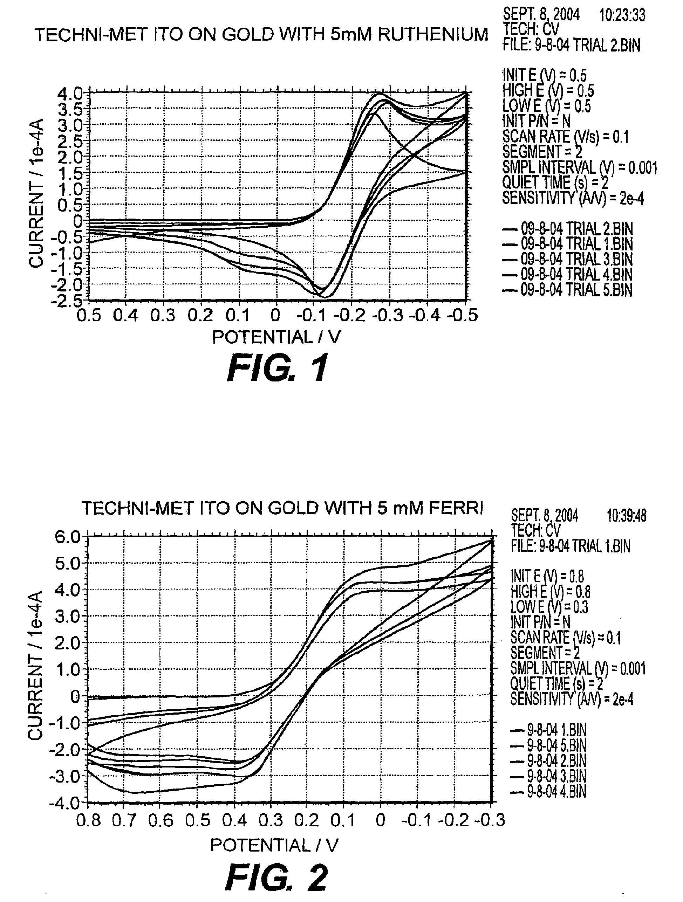

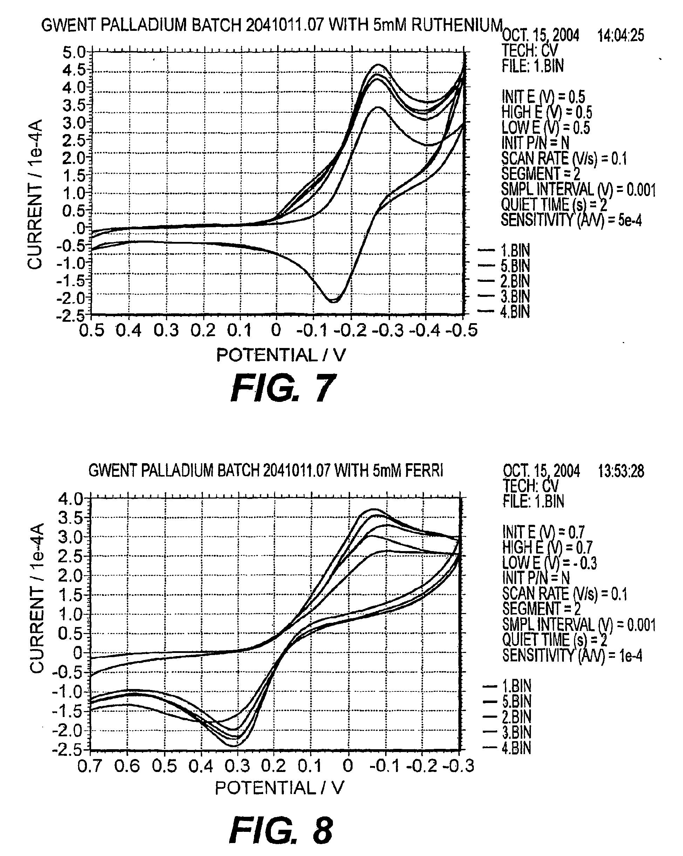

Electrochemical data were obtained using the various biosensors described herein. The biosensor "samples" were placed in an electrochemical cell as the working electrode. Pt wire was used as the counter electrode, and Ag/AgCl electrode was used as the reference. The solution of interest was placed over the working electrode with the counter and reference electrodes being immersed in it. A potential was applied using a CHI 600A electrochemical analyzer within the potential limits specified on Figs. 1-8, and the resulting current was recorded.

The relevant parameters of interest in cyclic voltammograms include: peak current, peak potential and peak separation. Larger oxidation peak current indicates a larger signal that can be obtained using a particular mediator, at a specified mediator concentration and scan rate. Peak separation is an indication of the electron-transfer kinetics; for an ideal system, it should be 60 mV/n, where n is the number of electrons exchanged between the redox probe and the electrode surface.

### Analysis of the Electrodes Physical Properties

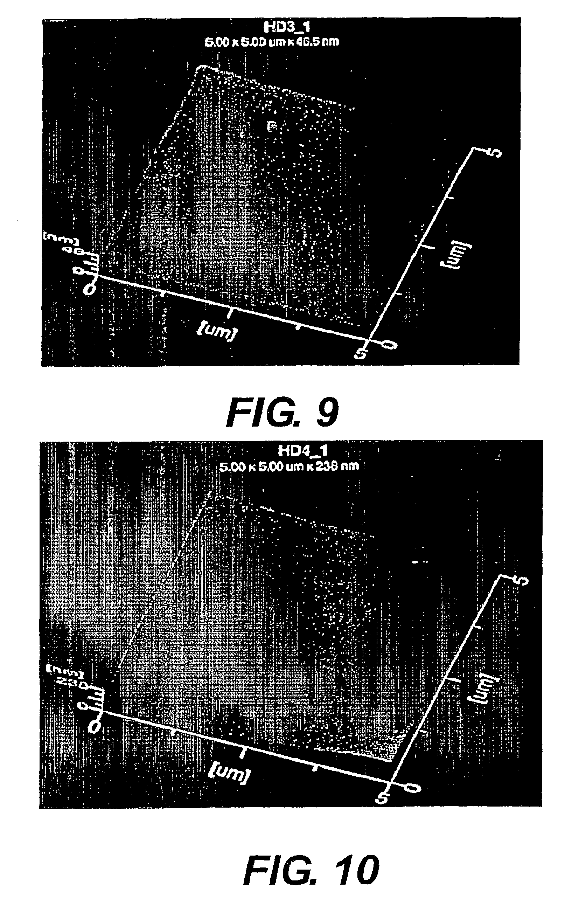

The process of electron transfer is a function of the interface between the electrode and electrolyte. Thus, the redox kinetics are influenced by the physical properties of the electrode, including surface area and density of active electron-transfer sites. To analyze such properties, including morphology and surface roughness, atomic force microscopy was used. Typical morphologies for electrode materials are shown in Figs. 9 and 10, for IZO/Au films and carbon films, respectively.

Surface roughness of the electrodes typically increased with increasing chamber pressures during film deposition. While not wishing to be bound by any theory, it is believed that higher pressures have the effect of thermalizing the ions incident on the surface, which results in greater surface roughness.

Generally, increased surface roughness resulting from increased chamber pressure during film deposition leads to an increase in charging current. However, there is not typically a significant difference in peak separation and peak current with such rougher surfaces.

### Evaluation of a Glucose Biosensor

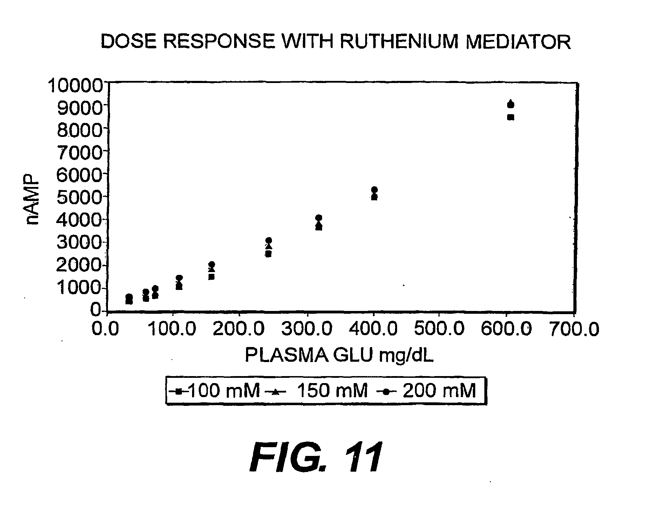

The resulting biosensor was also evaluated as a function of analyte and mediator concentration. In particular, dose response and glucose values were measured as a function of glucose concentration and excitation voltage, respectively, for a ruthenium mediator. For example, Fig. 11 shows the dose response as a function of glucose concentration for biosensors using mediators comprising 100, 150, and 200mM ruthenium hexaamine (III) trichloride. At 150mM and 200mM, the biosensor response was nearly identical, especially at higher glucose levels.

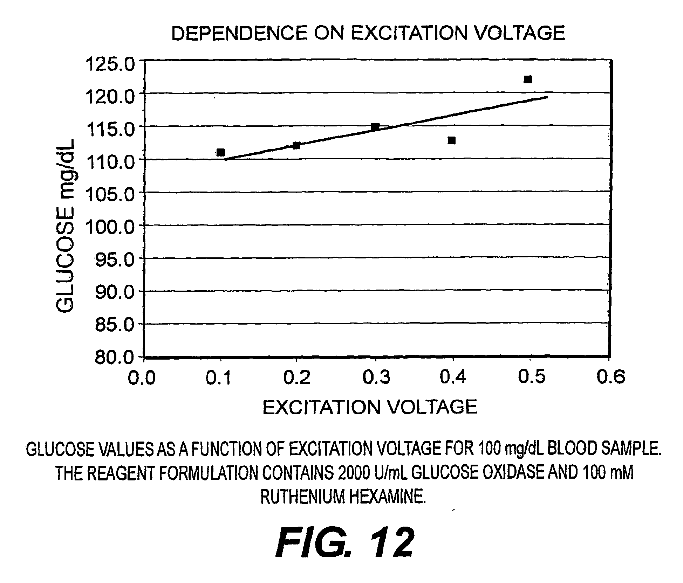

Fig. 12 shows glucose values as a function of excitation voltage for a 100 mg/dL blood sample using a reagent formulation comprising a glucose oxidase and a ruthenium hexaamine (III) trichloride mediator. In particular, a biosensor comprising a reagent formulation containing 2000 U/mL glucose oxidase and 100mM ruthenium hexaamine (III) trichloride was evaluated.

### Shortened Test Time

Test times for the resulting biosensors were also analyzed as a function of the mediator. It was discovered that strips made using a Ru mediator showed no degradation of precision, stability or temperature sensitivity even at zero incubation time. Incubation time is defined as the time between sample detection and applying excitation voltage.

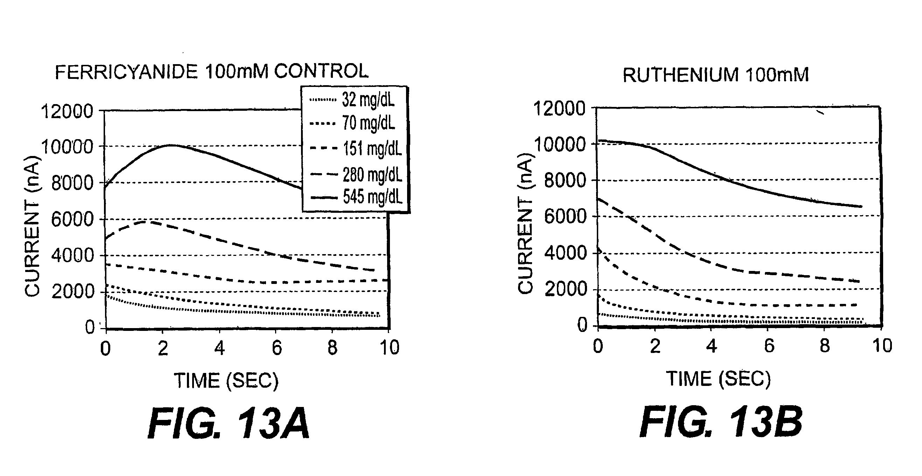

As shown in Fig. 13, strips made with a ruthenium mediator showed better kinetics with a zero-wait than strips made with a ferricyanide mediator. In particular, Fig. 13(b) shows that there is no rise in the ruthenium signal early in the kinetics as there is for the ferricyanide signal 13(a). For this reason, the precision is worse in the ferricyanide strips early in the response since the early (rising) phase of the ferricyanide kinetics is more variable with high glucose samples than it is for ruthenium.

The present disclosure is further illuminated by the following non-limiting examples, which are intended to be purely exemplary of the invention.

### Example 1: Semiconductor Electrodes

This example summarizes the results of physical and electrochemical analysis on various semiconducting electrodes according to the present disclosure. Tin doped indium oxide ("ITO") and zinc doped indium oxide ("IZO") films were deposited by sputtering (dc Magnetron Sputter) using 90/10 indium oxide/tin or zinc oxide targets. In order to achieve high conductivity of the film in a cost effective manner, ITO and IZO were deposited on a thin layer of Au (60-100 A).

The semiconducting films of ITO were sputter deposited at room temperature. X-ray diffraction analysis of the as-sputtered films showed they were amorphous and electrochemical characterization of the amorphous samples showed voltammograms with poor or non-existent signals, indicating poor electron transfer properties. In contrast, after annealing the amorphous films at 250°C for 1 hour, the films showed strong crystalline peaks, indicating a polycrystalline film was formed.

Electrochemical properties performed on the polycrystalline samples showed voltammograms with strong signals, indicating more efficient electron transfer than the amorphous samples.

The polycrystalline and amorphous samples were each tested with a ruthenium hexaamine electron mediator and with a ferricyanide electron mediator. These analytes were chosen because of their charge differences and the fact that their electrochemical behavior on a variety of electrode materials is well-documented and understood.

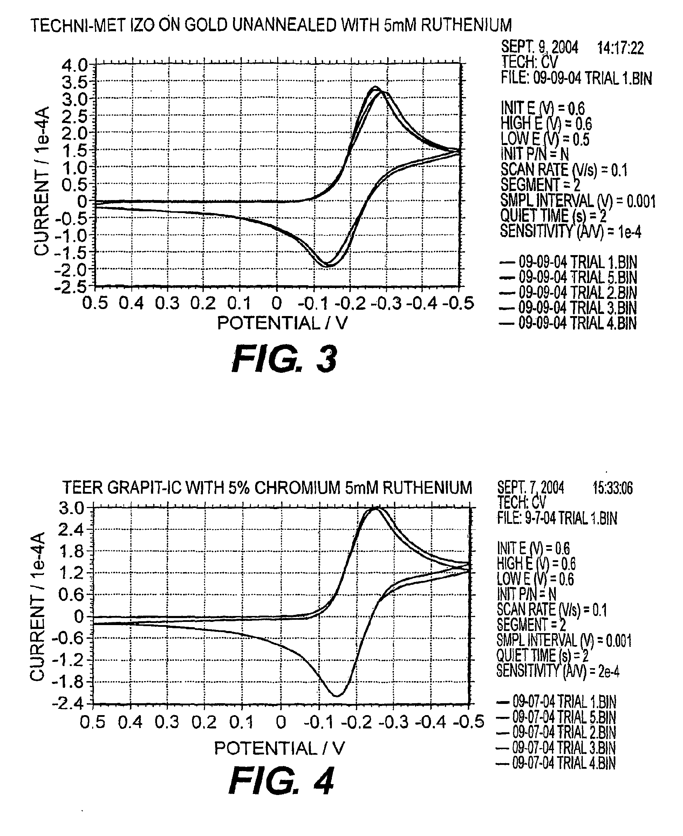

As shown in Figs. 1 and 2, the ITO film performed well with both ruthenium hexaamine and ferricyanide, with a more ideal voltammogram associated with the use of the ruthenium hexaamine (Fig. 1) when compared to ferricyanide (Fig. 2). In addition, as shown in Fig. 3, the cyclic voltammogram associated with the use of IZO with a ruthenium hexaamine mediator showed excellent electron transfer kinetics.

### Example 2: Sputtered Carbon Electrodes

This example summarizes the results of physical and electrochemical analysis on sputtered carbon electrodes according to the present disclosure.

A thin film of carbon was sputtered (dc Magnetron) onto a polyethylene terephthalate (PET) substrate. The sputtering technique involved sputtering Cr as a seed layer and gradually decreasing the power on the Cr target while simultaneously increasing the power on the carbon target. The resulting film was 0.5 um thick and had the conductivity of 8 ohms/square. Because Cr was first sputtered as the seed layer, the resulting film contained about 5% Cr. Fig. 10 shows an AFM of a carbon film electrode.

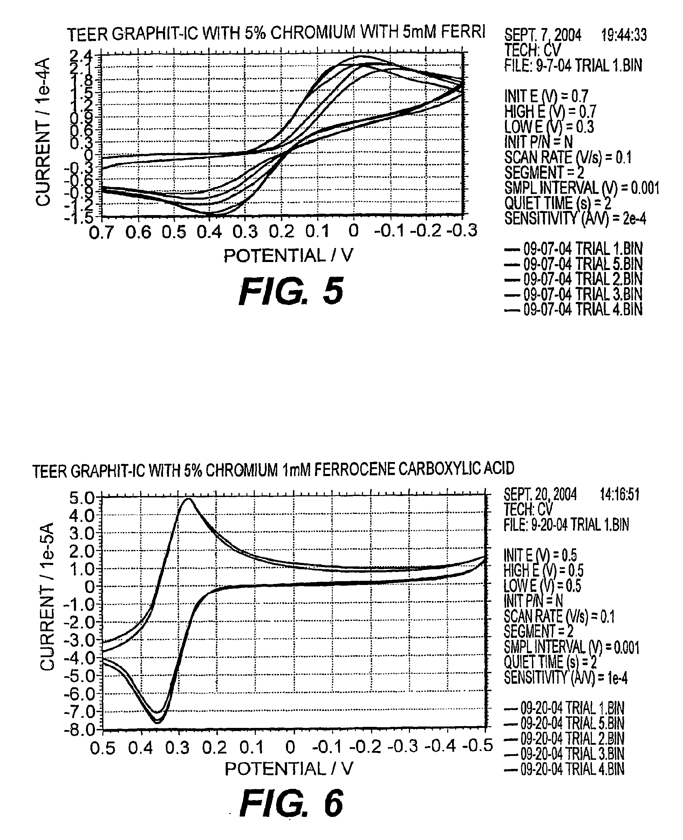

As shown in Fig. 4, cyclic voltammograms of ruthenium hexaamine obtained on the previously described carbon films show excellent electron-transfer kinetics. The performance of the carbon films with ferricyanide as the electron mediator is shown in Fig. 5, which electrochemical properties are acceptable for use in biosensor applications. In addition, as shown in Fig. 6, the carbon film gave ideal cyclic voltammetric response with ferrocene carboxylic acid as the electron mediator.

### Example 3: Performance Of Zinc-Doped Indium Oxide Electrodes In Glucose Biosensors

Zinc-doped indium oxide (IZO) electrodes were used to assemble glucose sensor strips with the goal of evaluating sensor performance. IZO film was sputtered on top of a 10 nm thick gold layer to yield an overall conductivity of 25 ohms/square. Electrodes were formed by laser ablation with spacing of at least 100 µm between each electrode. Sensors were assembled using two different chemistry solutions that contained either glucose oxidase (GO) or glucose dehydrogenase (GDH). In all cases, ruthenium hexaamine was used as the mediator. In the case of GDH, sucrose was added to the formulation as a stabilizer for the enzyme. Sensors assembled using gold electrodes were tested in parallel with the sensors made with IZO electrodes.

A summary of the data on gold and IZO sensors having a chemistry solution that contained glucose oxidase (GO) is shown in Table 2.

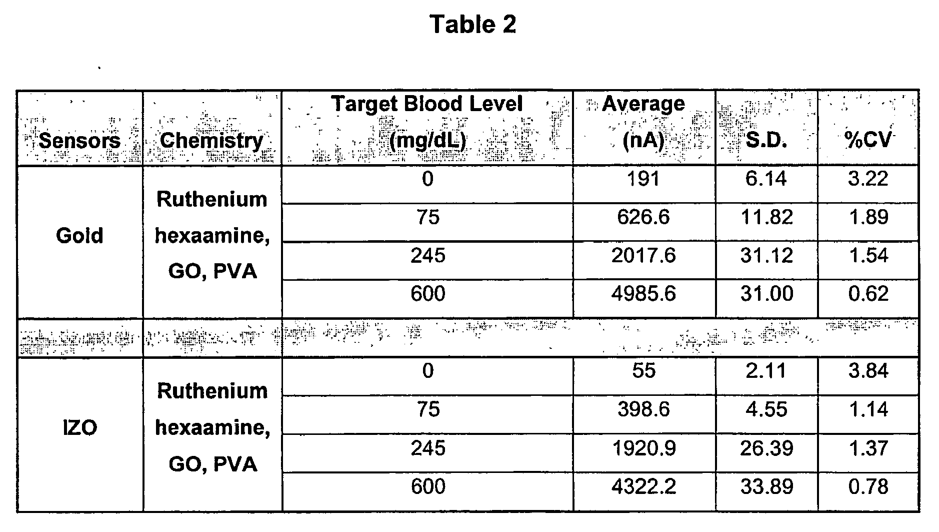

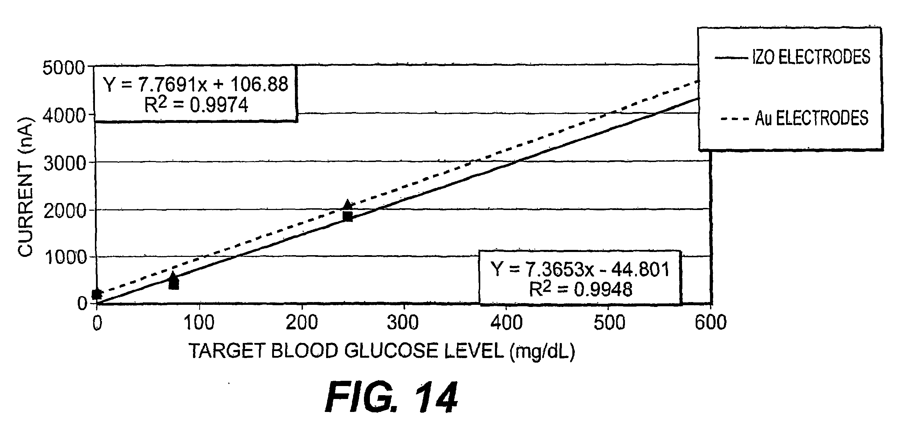

The results of Table 2 are also graphically shown in Fig. 14. These results demonstrate that IZO sensors gave comparable signal and precision to gold sensors. In addition, IZO sensors had a much lower background signal than gold sensors, which might lead to improved stability and linearity of the calibration curves. Lower background signal is clearly observed in the dose response graph (Fig. 14), which is linear for both types of sensors and shows the offset for the IZO sensors that is derived from the lower background.

A summary of the data on gold and IZO sensors having a chemistry solution that contained glucose dehydrogenase (GDH) is shown in Table 3.

**Table 3**

| **Sensors** | **Chemistry** | **Target Blood Level (mg/dL)** | **Average (nA)** | **%CV** |
|---|---|---|---|---|
| **Gold** | **Ruthenium hexamine, GDH, PVA, sucrose** | 0 | 241.2 | 8.10 |
| | | 75 | 1099.4 | 5.50 |
| | | 245 | 2861.8 | 4.95 |
| | | 600 | 5888 | 5.11 |
| | | | | |
| **IZO** | **Ruthenium hexamine, GDH, PVA, sucrose** | 0 | 35.5 | 18.10 |
| | | 75 | 942.1 | 4.93 |
| | | 245 | 3010.2 | 3.82 |
| | | 600 | 5677.3 | 2.71 |

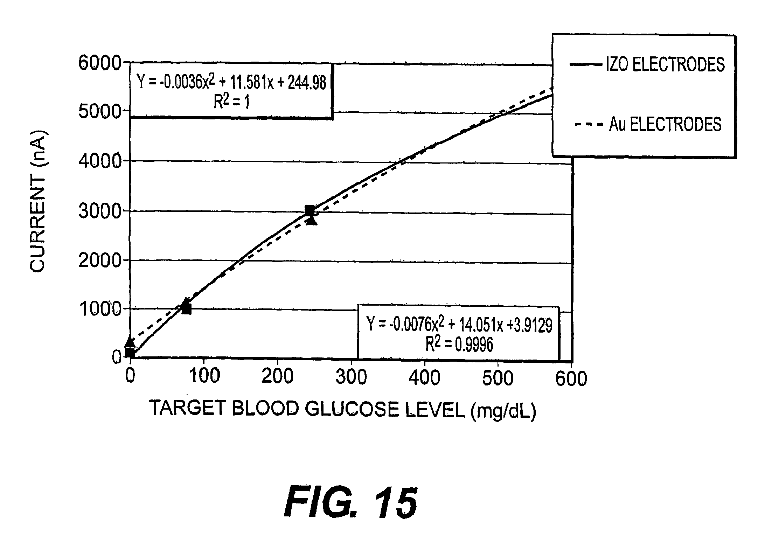

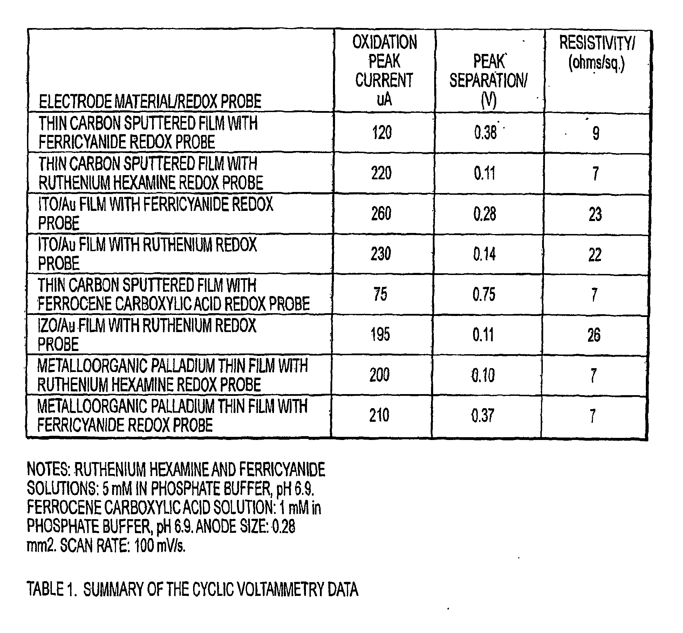

The results of Table 3 are also graphically shown in Fig. 15. These results demonstrate that the difference in the background signal between gold and IZO electrodes was even more pronounced for GDH-based chemistry than for GO-based chemistry.

In addition to offering performance benefits, IZO may be more compatible with different meter connector designs, since it is much more scratch resistant than gold.

### Example 4: Moisture Sensitivity Analysis

This example illustrates the significant improvements of moisture sensitivity for reagent formulations containing ruthenium hexaamine (III) trichloride as mediator.

Separate sensor strips were prepared with reagent formulations containing either (1) potassium ferricyanide as mediator or (2) ruthenium hexaamine (III) trichloride as mediator. The sensor strips were stored in desiccated vials. One set of vials have intentionally punctured holes on the lids and were exposed to high moisture environment (30°C/80% RH) for 5 days. Another set of vials were kept intact and stored normally at room temperature as a control.

After 5-day incubation in high humidity, strips were tested using glucose control and blood sample with 110 mg/dL glucose. The test results are summarized in Table 4 and Table 5.

**Table 4 Glucose Control Test Results**

| | Ferricyanide formulation | Ruthenium formulation | Ruthenium formulation with 0.1 % Natrosol 250L |
|---|---|---|---|
| Control strips stored at RT | 128 | 195 | 231 |
| Strips exposed to 30 °C, 80% RH for 5 days | 293 | 201 | 234 |

As shown in Table 4, sensors made with ferricyanide as mediator showed an increase of 165 mg/dL in glucose recovery value, while sensors made with ruthenium as mediator showed an increase of only 3 - 6 mg/dL. It is important to note that glucose recovery value may be different for different reagent formulation when tested with the same glucose control and only the change of glucose recovery value is indicative of the sensitivity to environment. Thus, the ruthenium hexaamine (III) trichloride mediator was found to be much less sensitive to moisture exposure when compared to ferricyanide which is used as mediator for many glucose test strips currently on the market.

**Table 5 Blood Sample Test Results**

| | Ferricyanide formulation | Ruthenium formulation | Ruthenium formulation with 0.1% Natrosol 250L |
|---|---|---|---|
| Control strips stored at RT | 109 | 117 | 120 |
| Strips exposed to 30 °C, 80% RH for 5 days | 319 | 136 | 130 |

As shown in Table 5, similar results were observed when strips were tested with blood samples. Sensors made using ruthenium hexaamine (III) trichloride as mediator were far less sensitive to moisture than sensors made using ferricyanide as mediator. For example, sensors containing ruthenium hexaamine (III) trichloride mediator showed an increase of about 10-20 mg/dL, while sensors containing ferricyanide as mediator increased about 210 mg/dL.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention.

## Claims

1. A biosensor for measuring analyte in a fluid by applying an excitation potential across the electrodes and measuring the resulting current, said biosensor comprising:
at least one semiconducting electrode; and
a reaction reagent system comprising a ruthenium hexaamine (III) trichloride containing electron mediator and an oxidation-reduction enzyme specific for said analyte.

2. The biosensor of claim 1, wherein the at least one semiconducting electrode comprises a material chosen from tin oxide, indium oxide, titanium dioxide, manganese oxide, iron oxide, and zinc oxide.

3. The biosensor of claim 2, wherein the at least one semiconducting electrode comprises zinc oxide doped with indium, tin oxide doped with indium, indium oxide doped with zinc, or indium oxide doped with tin.

4. The biosensor of claim 1, wherein the at least one semiconducting electrode comprises an allotrope of carbon doped with boron, nitrogen, or phosphorous.

5. The biosensor of claim 1, wherein the analyte is chosen from glucose, cholesterol, lactate, acetoacetic acid (ketone bodies), theophylline, and hemoglobin A1c.

6. The biosensor of claim 5, wherein the analyte comprises glucose and the at least one oxidation-reduction enzyme specific for the analyte is chosen from glucose oxidase, PQQ-dependent glucose dehydrogenase and NAD-dependent glucose dehydrogenase.

7. The biosensor of claim 1, wherein the fluid comprises blood and the reaction reagent system comprises a red blood cell binding agent for capturing red blood cells from the fluid, said red blood cell binding agent comprising lectins.

8. The biosensor of claim 1, wherein the reaction reagent system further comprises at least one buffer material comprising potassium phosphate.

9. The biosensor of claim 1, wherein the reaction reagent system further comprises at least one surfactant chosen from non-ionic, anionic, and zwitterionic surfactants.

10. The biosensor of claim 1, wherein the reaction reagent system further comprises at least one polymeric binder chosen from hydroxypropyl-methyl cellulose, sodium alginate, microcrystalline cellulose, polyethylene oxide, hydroxyethylcellulose, polypyrrolidone, PEG, and polyvinyl alcohol.

11. The biosensor of claim 1, wherein the reaction reagent system comprises 0.01 to 0.3% of a non-ionic surfactant and 0.1 to 3%, of a polymeric binder material.

12. The biosensor of claim 11, wherein the reaction reagent system comprises 0.05 to 0.25% of an alkyl phenoxy polyethoxy ethanol and 0.5 to 2.0% of polyvinyl alcohol.

13. A biosensor for measuring analyte in a fluid by applying an excitation potential across the electrodes and measuring the resulting current, said biosensor comprising:
at least one conducting electrode; and
a reaction reagent system comprising a ruthenium hexaamine (III) trichloride containing electron mediator and an oxidation-reduction enzyme specific for said analyte.

14. The biosensor of claim 13, wherein the at least one conducting electrode comprises a metal chosen from or derived from gold, platinum, rhodium, palladium, silver, iridium, carbon, steel, metallorganics, and mixtures thereof.

15. The biosensor of claim 14, wherein the at least one carbon electrode further comprising Cr.

16. The biosensor of claim 13, wherein the analyte is chosen from glucose, cholesterol, lactate, acetoacetic acid (ketone bodies), theophylline, and hemoglobin A1c.

17. The biosensor of claim 16, wherein the analyte comprises glucose and the at least one oxidation-reduction enzyme specific for the analyte is chosen from glucose oxidase, PQQ-dependent glucose dehydrogenase and NAD-dependent glucose dehydrogenase.

18. The biosensor of claim 13, wherein the fluid comprises blood and the reaction reagent system comprises a red blood cell binding agent for capturing red blood cells from the fluid, said red blood cell binding agent comprising lectins.

19. The biosensor of claim 13, wherein the reaction reagent system further comprises at least one buffer material comprising potassium phosphate.

20. The biosensor of claim 13, wherein the reaction reagent system further comprises at least one surfactant chosen from non-ionic, anionic, and zwitterionic surfactants.

21. The biosensor of claim 13, wherein the reaction reagent system further comprises at least one polymeric binder chosen hydroxypropyl-methyl cellulose, sodium alginate, microcystalline cellulose, polyethylene oxide, hydroxyethylcellulose, polypyrrolidone, PEG, and polyvinyl alcohol.

22. The biosensor of claim 13, wherein the reaction reagent system comprises 0.01 to 0.3% of a non-ionic surfactant and 0.1 to 3%, of a polymeric binder material.

23. The biosensor of claim 22, wherein the reaction reagent system comprises 0.05 to 0.25% of an alkyl phenoxy polyethoxy ethanol and 0.5 to 2.0% of polyvinyl alcohol.

## Patentansprüche

1. Biosensor zum Messen eines Analyten in einem Fluid durch Anlegen eines Anregungspotentials über die Elektroden und Messen des resultierenden Stroms, wobei der Biosensor umfasst:
wenigstens eine Halbleiterelektrode; und
ein Reaktionsreagenssystem, das ein Rutheniumhexamin-(III-)Trichiorid, das ein Elektronenzwischenreagens enthält, und ein für den Analyten spezifisches Oxidations-Reduktions-Enzym umfasst.

2. Biosensor nach Anspruch 1, bei dem die wenigstens eine Halbleiterelektrode ein Material umfasst, das aus Zinnoxid, Indiumoxid, Titandioxid, Manganoxid, Eisenoxid und Zinkoxid ausgewählt ist.

3. Biosensor nach Anspruch 2, bei dem die wenigstens eine Halbleiterelektrode Zinkoxid, das mit Indium dotiert ist, Zinnoxid, das mit Indium dotiert ist, Indiumoxid, das mit Zink dotiert ist, oder Indiumoxid, das mit Zinn dotiert ist, umfasst.

4. Biosensor nach Anspruch 1, bei dem die wenigstens eine Halbleiterelektrode ein Allotrop von Kohlenstoff umfasst, das mit Bor, Stickstoff oder Phosphor dotiert ist.

5. Biosensor nach Anspruch 1, bei dem der Analyt aus Glucose, Cholesterin, Lactat, Acetessigsäure (Ketonkörper), Theophyllin und Hämoglobin A1c ausgewählt ist.

6. Biosensor nach Anspruch 5, bei dem der Analyt Glucose umfasst und das wenigstens eine für den Analyten spezifische Oxidations-Reduktions-Enzym aus Glucose-Oxidase, von PQQ abhängiger Gluoose-Dehydrogenase und von NAD abhängiger Glucose-Dehydrogenase ausgewählt ist.

7. Biosensor nach Anspruch 1, bei dem das Fluid Blut umfasst und das Reaktionsreagenssystem ein Mittel zum Binden roter Blutkörperchen zum Erfassen roter Blutkörperchen aus dem Fluid umfasst, wobei das Mittel zum Binden roter Blutkörperchen Lectine umfasst.

8. Biosensor nach Anspruch 1, bei dem das Reaktionsreagenssystem ferner wenigstens ein Puffermaterial umfasst, das Kaliumphosphat umfasst.

9. Biosensor nach Anspruch 1, bei dem das Reaktionsreagenssystem ferner wenigstens einen grenzflächenaktiven Stoff umfasst, der aus nichtionischen, anionischen und zwitterionischen grenzflächenaktiven Stoffen ausgewählt ist.

10. Biosensor nach Anspruch 1, bei dem das Reaktionsreagenssystem ferner wenigstens ein Polymerbindemittel umfasst, das aus Hydroxypropylmethylcellulose, Natriumalginat, mikrokristalliner Cellulose, Polyethylenoxid, Hydroxyethylcellulose, Polypyrrolidon, PEG und Polyvinylalkohol ausgewählt ist.

11. Biosensor nach Anspruch 1, bei dem das Reaktionsreagenssystem 0,01 bis 0,3 % eines nichtionischen grenzflächenaktiven Stoffs und 0,1 bis 3 % eines Polymerbindematerials umfasst.

12. Biosensor nach Anspruch 11, bei dem das Reaktionsreagenssystem 0,05 bis 0,25 % eines Alkylphenoxypolyethoxyethanols und 0,5 bis 2,0 % Polyvinylalkohol umfasst.

13. Biosensor zum Messen eines Analyten in einem Fluid durch Anlegen eines Anregungspotentials über die Elektroden und Messen des resultierenden Stroms, wobei der Biosensor umfasst:
wenigstens eine leitende Elektrode; und
ein Reaktionsreagenssystem, das ein Rutheniumhexamin-(III-)Trichlorid, das ein Elektronenzwischenreagens enthält, und ein für den Analyten spezifisches Oxidations-Reduktions-Enzym umfasst.

14. Biosensor nach Anspruch 13, bei dem die wenigstens eine leitende Elektrode ein Metall umfasst, das aus Gold, Platin, Rhodium, Palladium, Silber, Iridium, Kohlenstoff, Stahl, metallorganischen Verbindungen und Gemischen davon ausgewählt oder davon abgeleitet ist.

15. Biosensor nach Anspruch 14, bei dem die wenigstens eine Kohlenstoffelektrode ferner Cr umfasst.

16. Biosensor nach Anspruch 13, bei dem der Analyt aus Glucose, Cholesterin, Lactat, Acetessigsäure (Ketonkörper), Theophyllin und Hämoglobin A1c ausgewählt ist.

17. Biosensor nach Anspruch 16, bei dem der Analyt Glucose umfasst und das wenigstens eine für den Analyten spezifische Oxidations-Reduktions-Enzym aus Glucose-Oxidase, von PQQ abhängiger Gluoose-Dehydrogenase und von NAD abhängiger Glucose-Dehydrogenase ausgewählt ist.

18. Biosensor nach Anspruch 13, bei dem das Fluid Blut umfasst und das Reaktionsreagenssystem ein Mittel zum Binden roter Blutkörperchen zum Erfassen roter Blutkörperchen aus dem Fluid umfasst, wobei das Mittel zum Binden roter Blutkörperchen Lectine umfasst.

19. Biosensor nach Anspruch 13, bei dem das Reaktionsreagenssystem ferner wenigstens ein Puffermaterial umfasst, das Kaliumphosphat umfasst.

20. Biosensor nach Anspruch 13, bei dem das Reaktionsreagenssystem ferner wenigstens einen grenzflächenaktiven Stoff umfasst, der aus nichtionischen, anionischen und zwitterionischen grenzflächenaktiven Stoffen ausgewählt ist.

21. Biosensor nach Anspruch 13, bei dem das Reaktionsreagenssystem ferner wenigstens ein Polymerbindemittel umfasst, das aus Hydroxypropylmethylcellulose, Natriumalginat, mikrokristalliner Cellulose, Polyethylenoxid, Hydroxyethylcellulose, Polypyrrolidon, PEG und Polyvinylalkohol ausgewählt ist.

22. Biosensor nach Anspruch 13, bei dem das Reaktionsreagenssystem 0,01 bis 0,3 % eines nichtionischen grenzflächenaktiven Stoffs und 0,1 bis 3 % eines Polymerbindematerials umfasst.

23. Biosensor nach Anspruch 22, bei dem das Reaktionsreagenssystem 0,05 bis 0,25 % eines Alkylphenoxypolyethoxyethanols und 0,5 bis 2,0 % Polyvinylalkohol umfasst.

## Revendications

1. Biocapteur pour la mesure d'un analyte dans un fluide par application d'un potentiel d'excitation entre les électrodes et mesure du courant résultant, ledit biocapteur comprenant :
au moins une électrode semi-conductrice ; et
un système réaction réactif comprenant un trichlorure de ruthénium hexamine (III) contenant un médiateur d'électrons et un enzyme d'oxydoréduction spécifique pour ledit analyte.

2. Biocapteur selon la revendication 1, dans lequel la au moins une électrode semi-conductrice comprend un matériau choisi parmi l'oxyde stannique, l'oxyde d'indium, le dioxyde de titane, l'oxyde de manganèse, l'oxyde de fer, et l'oxyde de zinc.

3. Biocapteur selon la revendication 2, dans lequel la au moins une électrode semi-conductrice comprend l'oxyde de zinc doté d'indium, l'oxyde stannique doté d'indium, l'oxyde d'indium doté de zinc ou l'oxyde d'indium doté d'étain.

4. Biocapteur selon la revendication 1, dans lequel la au moins une électrode semi-conductrice comprend un allotrope de carbone doté de bore, d'azote ou de phosphore.

5. Biocapteur selon la revendication 1, dans lequel l'analyte est choisi parmi le glucose, le cholestérol, le lactate, l'acide acétoacétique (des corps de cétone), la théophylline, et l'hémoglobine A1c.

6. Biocapteur selon la revendication 5, dans lequel l'analyte comprend le glucose et le au moins un enzyme d'oxydoréduction spécifique pour l'analyte est choisi parmi l'oxydase de glucose, la déshydrogénase de glucose dépendant de PQQ et la déshydrogénase de glucose dépendant de NAD.

7. Biocapteur selon la revendication 1, dans lequel le fluide comprend du sang et le système réaction réactif comprend un agent de liaison de cellules sanguines rouges pour la capture de cellules sanguines rouges du fluide, ledit agent de liaison de cellules sanguines rouges comprenant des lectines.

8. Biocapteur selon la revendication 1, dans lequel le système réaction réactif comprend en outre au moins un matériau tampon comprenant le phosphate de potassium.

9. Biocapteur selon la revendication 1, dans lequel le système réaction réactif comprend en outre au moins un surfactant choisi parmi des surfactants non ioniques, anioniques et zwitterionique.

10. Biocapteur selon la revendication 1, dans lequel le système réaction réactif comprend en outre un liant polymérique choisi parmi l'hydroxypropylméthylcellulose, l'alginate de sodium, la cellulose microcristalline, l'oxyde de polyéthylène, l'hydroxyéthylcellulose, la polypyrrolidone, le PEG, et l'alcool polyvinylique.

11. Biocapteur selon la revendication 1, dans lequel le système réaction réactif comprend 0,01 à 0,3% d'un surfactant non ionique et 0,1 à 3% d'un matériau liant polymérique.

12. Biocapteur selon la revendication 11, dans lequel le système réaction réactif comprend 0,05 à 0,25% d'un alkylphénoxypolyéthoxyéthanol et 0,5 à 2,0% d'alcool polyvinylique.

13. Biocapteur pour la mesure d'un analyte dans un fluide par application d'un potentiel d'excitation entre les électrodes et mesure du courant résultant, ledit biocapteur comprenant :
au moins une électrode conductrice ; et
un système réaction réactif comprenant un trichlorure de ruthénium hexamine (III) contenant un médiateur d'électrons et un enzyme d'oxydoréduction spécifique pour ledit analyte.

14. Biocapteur selon la revendication 13, dans lequel la au moins une électrode conductrice comprend un métal choisi parmi ou dérivé de l'or, le platine, le rhodium, le palladium, l'argent, l'iridium, le carbone, l'acier, des corps organométalliques, et des mélanges de ces derniers.

15. Biocapteur selon la revendication 14, dans lequel la au moins une électrode de carbone comprend en outre le Cr.

16. Biocapteur selon la revendication 13, dans lequel l'analyte est choisi parmi le glucose, le cholestérol, le lactate, l'acide acétoacétique (des corps de cétone), la théophylline, et l'hémoglobine A1c.

17. Biocapteur selon la revendication 16, dans lequel l'analyte comprend le glucose et le au moins un enzyme d'oxydoréduction spécifique pour l'analyte est choisi parmi l'oxydase de glucose, la déshydrogénase de glucose dépendant de PQQ et la déshydrogénase de glucose dépendant de NAD.

18. Biocapteur selon la revendication 13, dans lequel le fluide comprend du sang et le système réaction réactif comprend un agent de liaison de cellules sanguines rouges pour la capture de cellules sanguines rouges du fluide, ledit agent de liaison de cellules sanguines rouges comprenant des lectines.

19. Biocapteur selon la revendication 13, dans lequel le système réaction réactif comprend en outre au moins un matériau tampon comprenant le phosphate de potassium.

20. Biocapteur selon la revendication 13, dans lequel le système réaction réactif comprend en outre au moins un surfactant choisi parmi des surfactants non ioniques, anioniques et zwitterionique.

21. Biocapteur selon la revendication 13, dans lequel le système réaction réactif comprend en outre au moins un liant polymérique choisi parmi l'hydroxypropylméthylcellulose, l'alginate de sodium, la cellulose microcristalline, l'oxyde de polyéthylène, l'hydroxyéthylcellulose, la polypyrrolidone, le PEG, et l'alcool polyvinylique.

22. Biocapteur selon la revendication 13, dans lequel le système réaction réactif comprend 0,01 à 0,3% d'un surfactant non ionique et 0,1 à 3% d'un matériau liant polymérique.

23. Biocapteur selon la revendication 22, dans lequel le système réaction réactif comprend 0,05 à 0,25% d'un alkylphénoxypolyéthoxyéthanol et 0,5 à 2,0% d'alcool polyvinylique.
